(19) Europäisches Patentamt
European Patent Office
Office européen des brevets

(11) Veröffentlichungsnummer: **0 113 839**
**B1**

(12) # EUROPÄISCHE PATENTSCHRIFT

(45) Veröffentlichungstag der Patentschrift: **10.08.88**

(51) Int. Cl.⁴: **C 07 D 249/08**, C 07 D 231/12, A 01 N 43/64, A 01 N 43/56

(21) Anmeldenummer: 83111887.2

(22) Anmeldetag: 28.11.83

(54) **Azolyl-Acetophenonoximether und diese enthaltende Fungizide.**

(30) Priorität: 04.12.82 DE 3244985

(43) Veröffentlichungstag der Anmeldung: **25.07.84 Patentblatt 84/30**

(45) Bekanntmachung des Hinweises auf die Patenterteilung: **10.08.88 Patentblatt 88/32**

(84) Benannte Vertragsstaaten: **AT BE CH DE FR GB IT LI NL SE**

(56) Entgegenhaltungen:
EP-A-0 019 133
DE-A-2 657 578
DE-A-3 139 370

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch- Strasse 38, D-6700 Ludwigshafen (DE)**

(72) Erfinder: **Schulz, Guenter, Dr., An der Froschlache 3, D-6700 Ludwigshafen (DE)**
Erfinder: **Buschmann, Ernst, Dr., Georg- Ludwig-Krebs- Strasse 10, D-6700 Ludwigshafen (DE)**
Erfinder: **Zeeh, Bernd, Dr., Queichstrasse 5, D-6703 Limburgerhof (DE)**
Erfinder: **Sauter, Hubert, Dr., Neckarpromenade, D-6800 Mannheim 1 (DE)**
Erfinder: **Pommer, Ernst- Heinrich, Dr., Berliner Platz 7, D-6703 Limburgerhof (DE)**
Erfinder: **Ammermann, Eberhard, Dr., Sachsenstrasse 3, D-6700 Ludwigshafen (DE)**

**EP 0 113 839 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

POOR QUALITY

Beschreibung

Die vorliegende Erfindung betrifft neue wertvolle 1,2,4-Triazol-1-yl-acetophenonoximether, Verfahren zu ihrer Herstellung sowie ihre Verwendung als Fungizide.

Es ist bekannt, Azolylacetophenonderivate und Oximether als Fungizide zu verwenden (DE-A-2 431 407, DE-2 657 578, EP-A-19 133). Ihre Wirkung bei verschiedenen Pilzen und ihre Verträglichkeit bei verschiedenen Pflanzen ist jedoch unbefriedigend.

Es wurde nun gefunden, daß neue Azolylacetophenonoximether der Formel (I)

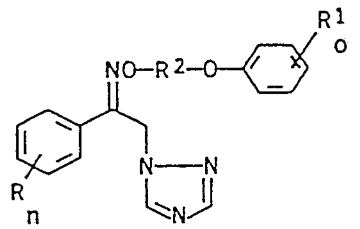

(I)

in welcher

R und $R^1$ unabhängig voneinander Wasserstoff, Halogen, $C_1$-$C_4$-Alkyl, $C_1$-$C_2$-Alkoxy, $C_1$-$C_2$-Alkylthio, $C_1$-$C_4$-Alkylsulfonyl, $C_1$-$C_4$-Halogenalkyl mit bis zu 5 Halogenatomen, Nitro, Cyano, Phenyl, Phenoxy oder durch Halogen, Cyano, Nitro oder $C_1$-$C_2$-Halogenalkyl mit bis zu 3 gleichen oder verschiedenen Halogenatomen substituiertes Phenyl oder Phenoxy bedeuten,

$R^2$ ein Diradikal der Formel $-(CH_2)m-$, der Formel $-(CH_2CH_2O-)_uCH_2CH_2-$, der Formel $-(CH_2)_v-CH{=}CH-(CH_2)_t-$ oder der Formel $-(CH_2)_v-C\equiv C-(CH_2)_t-$ bedeutet, wobei

n, o, u, v, t unabhängig voneinander 1, 2 oder 3, und

m 2 bis 8 bedeutet, und deren Salze und Metallkomplexverbindungen eine bessere fungizide Wirksamkeit und Pflanzenverträglichkeit haben als die oben genannten Fungizide.

In Formel (I) bedeuten beispielsweise:

R und $R^1$ unabhängig voneinander Halogen (Fluor, Chlor oder Brom), Nitro, Cyano, Alkyl oder Alkylsulfonyl mit jeweils 1 bis 4 Kohlenstoffatomen, Alkoxy- oder Alkylthio mit jeweils 1 bis 2 Kohlenstoffatomen, Halogenalkyl mit 1 bis 4 Kohlenstoffatomen und 1 bis 5 Halogenatomen, insbesondere mit 1 bis 2 Kohlenstoffatomen und 1 bis 3 gleichen oder verschiedenen Halogenatomen, wobei als Halogenatome vorzugsweise Fluor oder Chlor in Betracht kommen, und als Beispiel für Halogenalkyl Trifluotmethyl genannt wird. R und $R^1$ bedeuten außerdem unabhängig voneinander vorzugsweise gegebenenfalls einfach oder mehrfach (zwei- bis dreifach), gleichartig oder verschieden substituiertes Phenyl oder Phenoxy, wobei jeweils als Substituenten in Frage kommen: Halogen, insbesondere Fluor, Chlor oder Brom; Cyano, Nitro sowie Halogenalkyl mit 1 bis 2 Kohlenstoffatomen und 1 bis 3 gleichen oder verschiedenen Halogenatomen, wobei als Halogenatome vorzugsweise Fluor oder Chlor in Betracht kommen und als Beispiel für Halogenalkyl Trifluormethyl genannt wird.

$R^2$ steht in Formel (I) für ein Diradikal der Formel $-(CH_2)_m-$, wobei als Beispiele $-CH_2CH_2-$, $-(CH_2)_3-$, $-(CH_2)_4-$, $-(CH_2)_5-$, $-(CH_2)_6-$, $-(CH_2)_7-$ und $-(CH_2)_8-$ genannt werden.

Außerdem steht $R^2$ in Formel (I) für ein Diradikal mit einem, zwei oder drei Sauerstoffatomen, der Formel $(CH_2CH_2O)_uCH_2CH_2-$ wobei als Beispiele $-CH_2CH_2OCH_2CH_2-$, $-CH_2CH_2OCH_2CH_2OCH_2CH_2-$ und $(CH_2CH_2O)_3CH_2CH_2-$ genannt werden.

Außerdem steht $R^2$ in Formel (I) für ein Diradikal mit einer Doppel- oder einer Dreifachbindung der Formel $-(CH_2)_v-CH{=}CH-(CH_2)_t-$ oder der Formel $-(CH_2)_v-C\equiv C-(CH_2)_t-$. Als Beispiele werden $-CH_2-CH{=}CH-CH_2-$, $-(CH_2)_2-CH{=}CH-(CH_2)_2-$, $-(CH_2)_3-CH{=}CH-(CH_2)_3-$, $-CH_2-C\equiv C-CH_2-$, $-(CH_2)_2-C\equiv C-(CH_2)_2-$ und $-(CH_2)_3-C\equiv C-(CH_2)_3-$, genannt.

m steht für die ganzen Zahlen von 2 bis 8, z. B. die ganzen Zahlen 2, 3, 4, 5, 6, 7 und 8.

Als Salze der Azolylacetophenonoximether der Formel (I) seien beispielsweise die Hydrochloride, Hydrobromide, Sulfate, Phosphate und p-Toluol-sulfonate genannt.

Metallkomplexe sind beispielsweise die Addukte von Zink oder Kupfer, z. B. 1/2 $CuCl_2$ oder 1/2 $ZnCl_2$ an eine Verbindung der Formel (I).

Weiterhin wurde gefunden, daß man die neuen 1,2,4-Triazol-1-yl-acetophenonoximether der Formel (I), beispielsweise erhält, wenn man, wie in den folgenden Schemata angegeben vorgeht.

Schema A)

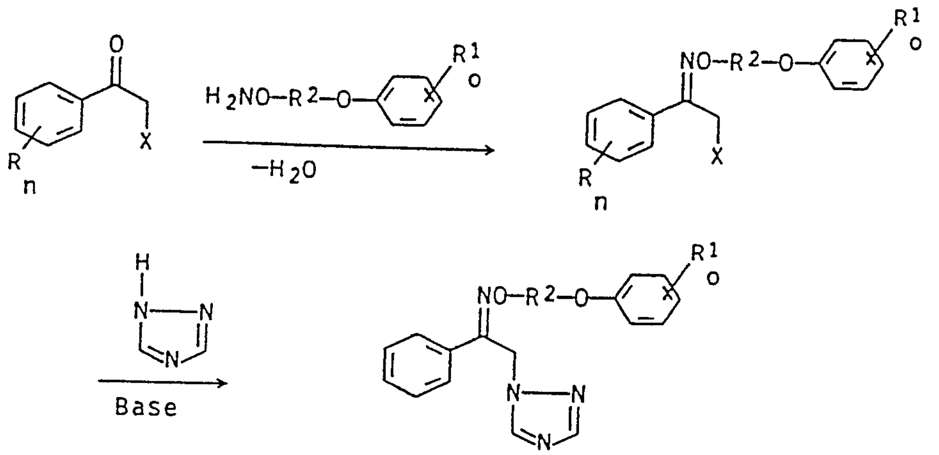


Schema B)

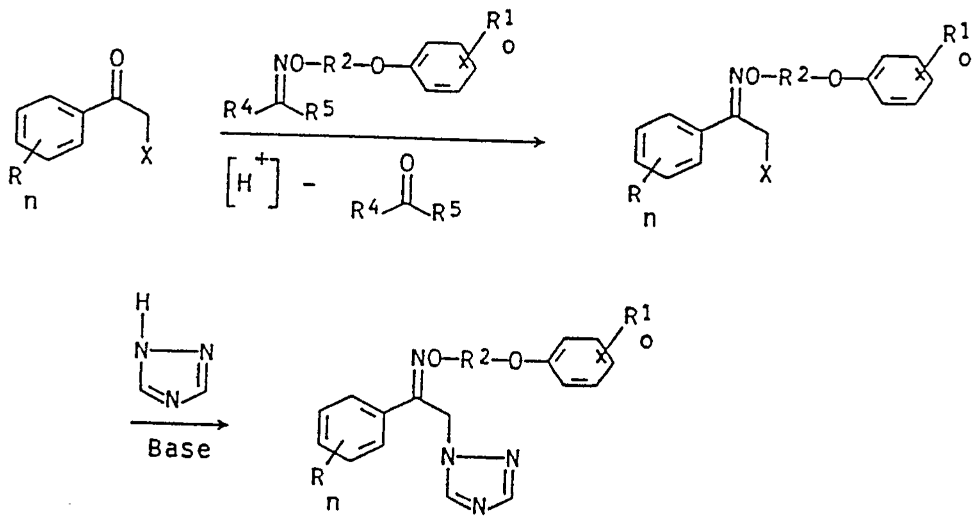


Schema C)

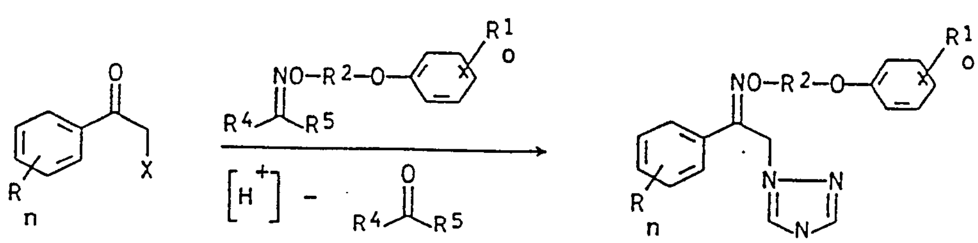

Schema D)

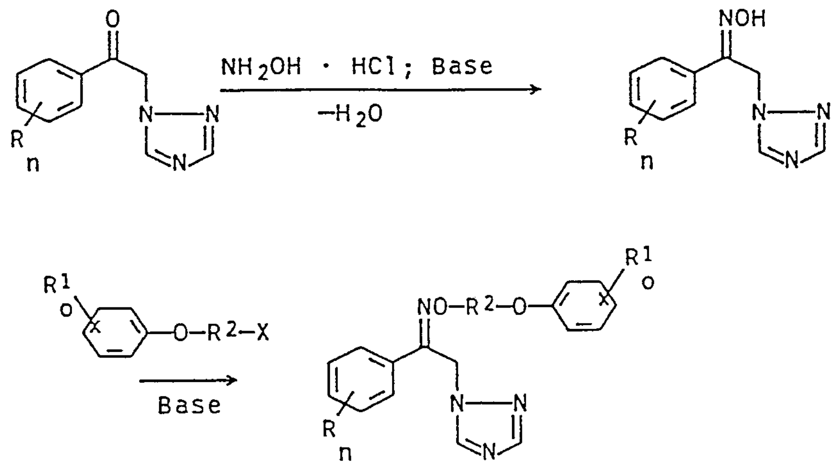


Schema E)

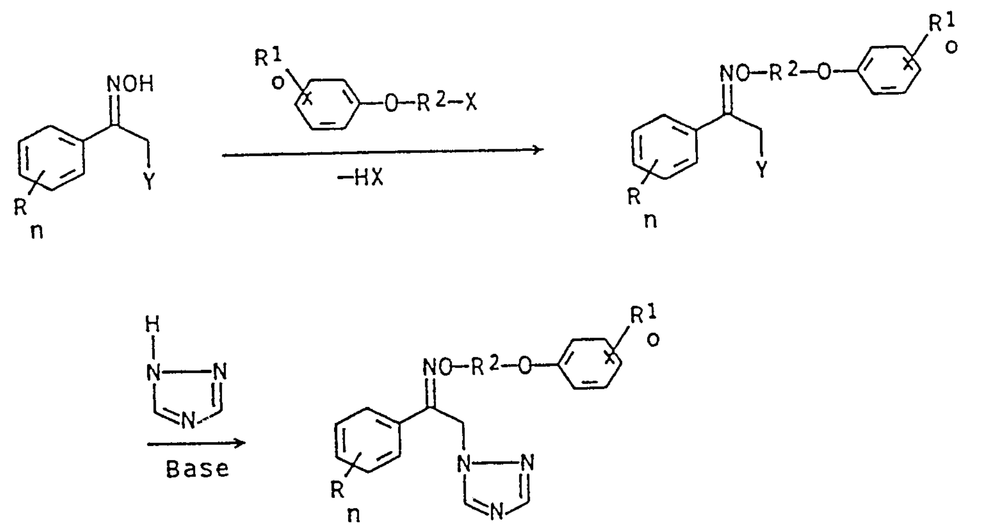


Die Umsetzung gemäß Schema B wird bevorzugt.

In den Schemata A bis E haben R, $R^1$, $R^2$, n und o die oben angegebene Bedeutung.

$R^4$ und $R^5$ bedeuten gleiche oder ungleiche Alkylreste mit 1 bis 5 Kohlenstoffatomen oder bilden einen Carbocyclus mit 5 bis 7 Kohlenstoffatomen.

X und Y steht für Cl, Br, J, Tosyl oder Mesyl. In der Formel

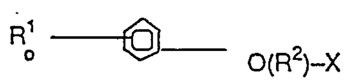

kann X auch den Sulfatrest bedeuten.

Die Ausgangsstoffe für die in den Schemata angegebenen Synthesewege sind bekannt (DE-A-2 431 407, DE-A-2 723 942) oder können analog zu bekannten Methoden dargestellt werden.

Die neuen 1,2,4-Triazolylacetophenonoximether C):liegen aufgrund der C=N-Doppelbindung als geometrische Isomere vor (E, Z-Formen). Durch geeignete Reaktionsführung, durch nachträgliche Isomerisierung oder durch Auftrennung der Isomerengemische kann man zu reinen E- oder Z-Formen gelangen, die ebenso wie ihre Mischungen von der vorliegenden Erfindung umfaßt werden und deren fungizide Wirkung unterschiedlich sein kann. Die Isomerengemische und auch

die reinen Isomeren haben z. T. ölige Konsistenz. Man kann sie durch Umsetzung mit Säuren oder Metallsalzen in ihre obengenannten Salze oder Metallkomplexe überführen, die meist kristallin sind und ausgezeichnete fungizide Wirksamkeit und gute Pflanzenverträglichkeit besitzen.

Das folgende Beispiel erläutert das Herstellungsverfahren:

**Herstellungsbeispiel**

2,4-Dichlor-ω-[1,2,4-triazolyl-(1)]-acetophenon-oxim-0-2-(4-chlorphenoxy)ethylether, E- und Z-Isomeres

23 g (0,085 mol) 2,4-Dichlor-ω-[1,2,4-triazolyl-(1)]-acetophenonoxim und 20 g 2-[4-Chlorphenoxy]-bromethan werden in 100 ml Toluol mit 7,1 g gepulverter KOH und 5,8 g Triethylbenzylammoniumchlorid C):bei 80 bis 100°C 1 h lang kräftig gerührt. Man dampft ein, nimmt in Dichlormethan (250 ml) auf, wäscht dreimal mit je 100 ml Wasser, trocknet die organische Phase mit Natriumsulfat und dampft ein. Chromatographie an Kieselgel mit Dichlormethan als Elutionsmittel liefert 6 g des Z-Isomeren (Wirkstoff Nr. 23) und 4 g des E-Isomeren des 2,4-Dichlor-ω-[1,2,4-triazolyl-(1)]-acetophenon-oxim-0-2-(4-chlorphenoxy)-ethylethers (Wirkstoff Nr. 24). Das Z-Isomere wird zuerst eluiert.

Entsprechend können die in der folgenden Tabelle aufgeführten Verbindungen hergestellt werden. Dabei wird die Konfiguration, bei der der Oximsauerstoff und der C-Aromat auf der gleichen Seite der Oximdoppelbindung stehen mit E bezeichnet, während das entsprechende andere Isomere die Bezeichnung Z erhält.

Ts = Tosyl, Bu = Butyl, Me = Methyl.

Entsprechend wurden diejenigen in der folgenden Tabelle angegebenen Verbindungen der Formel (I) hergestellt, deren Schmelzpunkte (Fp) oder charakteristische Banden angegeben sind. Ihre Struktur wurde durch Elementaranalyse gesichert. Die Verbindungen, bei denen keine Angaben zur physikalischen Chemie gemacht sind, können auf die gleiche Art und Weise, wie bei den tatsächlich hergestellten Verbindungen beschrieben, erhalten werden; es kann erwartet werden, daß sie aufgrund ihrer ähnlichen Konstitution ähnliche Wirkungen wie die näher untersuchten Verbindungen haben.

**Tabelle**

A) Verbindungen der Formel (I) mit $R^2 = -(CH_2)_m-$:

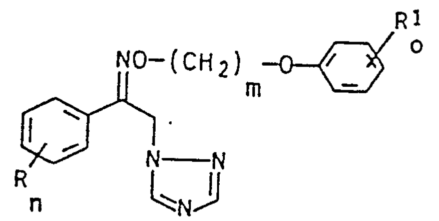

| Nr. | $R_n$ | $R_o^1$ | Salz | Konfiguration | m | Fp [°C] |
|---|---|---|---|---|---|---|
| 1 | H | H | -- | E/Z-Gemisch | 2 | |
| 2 | H | H | -- | E/Z-Gemisch | 4 | |
| 3 | H | H | -- | E/Z-Gemisch | 6 | |
| 4 | H | H | -- | E/Z-Gemisch | 8 | |
| 5 | 4-Cl | H | -- | E/Z-Gemisch | 2 | 96-98 |
| 6 | 4-Br | H | -- | E/Z-Gemisch | 2 | 89-90 |
| 7 | 4-OMe | H | -- | E/Z-Gemisch | 2 | |
| 8 | 4-CN | H | -- | E/Z-Gemisch | 2 | |
| 9 | 4-$CF_3$ | H | -- | E/Z-Gemisch | 2 | |
| 10 | 4-$NO_2$ | H | -- | E/Z-Gemisch | 2 | |
| 11 | 4-$OC_6H_5$ | H | -- | E/Z-Gemisch | 2 | |
| 12 | 2,4-$Cl_2$ | H | .$HNO_3$ | E/Z-Gemisch | 2 | 96 |
| 13 | 2,4-$Cl_2$ | H | -- | Z | 3 | Öl |
| 14 | 2,4-$Cl_2$ | H | -- | E | 3 | Öl |
| 15 | 2,4-$Cl_2$ | H | -- | Z | 4 | Öl |
| 16 | 2,4-$Cl_2$ | H | -- | E | 4 | Öl |
| 17 | 2,4-$Cl_2$ | H | -- | Z | 5 | Öl |
| 18 | 2,4-$Cl_2$ | H | -- | E | 5 | Öl |
| 19 | 2,4-$Cl_2$ | H | -- | Z | 6 | |
| 20 | 2,4-$Cl_2$ | H | -- | E | 6 | |
| 21 | 2,4-$Cl_2$ | H | -- | E | 8 | |
| 22 | 2,4-$Cl_2$ | H | -- | E | 8 | |
| 23 | 2,4-$Cl_2$ | 4-Cl | -- | Z | 2 | 87-91 |

| | | | | | | |
|---|---|---|---|---|---|---|
| 24 | 2,4-$Cl_2$ | 4-Cl | -- | E | 2 | 58-61 |
| 25 | 2,4-$Cl_2$ | 4-Cl | .$HNO_3$ | E/Z-Gemisch | 2 | |
| 26 | 2,4-$Cl_2$ | 4-Cl | .$HNO_3$ | E/Z-Gemisch | 2 | |
| 27 | 2,4-$Cl_2$ | 4-Cl | .$H_2SO_4$ | E/Z-Gemisch | 2 | |
| 28 | 2,4-$Cl_2$ | 4-Cl | .$H_3PO_4$ | E/Z-Gemisch | 2 | |
| 29 | 2,4-$Cl_2$ | 4-Cl | .TsOH | E/Z-Gemisch | 2 | |
| 30 | 2,4-$Cl_2$ | 4-Cl | .1/2 $CuCl_2$ | E/Z-Gemisch | 2 | |
| 31 | 2,4-$Cl_2$ | 4-Cl | Tri. 1/2 $ZnCl_2$ | E/Z-Gemisch | 2 | |
| 32 | 2,4-$Cl_2$ | 4-Cl | -- | Z | 3 | Öl |
| 33 | 2,4-$Cl_2$ | 4-Cl | -- | E | 3 | Öl |
| 34 | 2,4-$Cl_2$ | 4-Cl | -- | Z | 4 | 65-71 |
| 35 | 2,4-$Cl_2$ | 4-Cl | -- | E | 4 | Öl |
| 36 | 2,4-$Cl_2$ | 4-Cl | -- | Z | 5 | Öl |
| 37 | 2,4-$Cl_2$ | 4-Cl | -- | E | 5 | 59-62 |
| 38 | 2,4-$Cl_2$ | 2,4-$Cl_2$ | -- | Z | 2 | 95-100 |
| 39 | 2,4-$Cl_2$ | 2,4-$Cl_2$ | -- | E | 2 | Öl |
| 40 | 2,4-$Cl_2$ | 2,4-$Cl_2$ | .$HNO_3$ | E/Z-Gemisch | 2 | 132 (Zers.) |
| 41 | 2,4-$Cl_2$ | 2,4,6-$Cl_3$ | .HCl | E/Z-Gemisch | 2 | |
| 42 | 2,4-$Cl_2$ | 2,4,6-$Cl_3$ | .1/2 $ZnCl_2$ | E/Z-Gemisch | 2 | |
| 43 | 2,4-$Cl_2$ | 2,4,6-$Cl_3$ | .1/2 $CuCl_2$ | E/Z-Gemisch | 2 | |
| 44 | 2,4-$Cl_2$ | 4-F | -- | Z | 2 | 66-70 |
| 45 | 2,4-$Cl_2$ | 4-F | -- | E | 2 | 73-77 |
| 46 | 2,4-$Cl_2$ | 4-CN | -- | Z | 2 | 96-100 |
| 47 | 2,4-$Cl_2$ | 4-CN | -- | E | 2 | |
| 48 | 2,4-$Cl_2$ | 4-tert.-Bu | .$HNO_3$ | E/Z-Gemisch | 2 | 117 |
| 49 | 2,4-$Cl_2$ | 4-OMe | -- | Z | 2 | Öl |
| 50 | 2,4-$Cl_2$ | 4-OMe | -- | E | 2 | |
| 51 | 2,4-$Cl_2$ | 4-SMe | -- | E/Z-Gemisch | 2 | |
| 52 | 2,4-$Cl_2$ | 4-Br | -- | E/Z-Gemisch | 2 | |
| 53 | 2,4-$Cl_2$ | 4-$NO_2$ | -- | E/Z-Gemisch | 2 | |
| 54 | 2,4-$Cl_2$ | 3-Cl | -- | Z | 2 | Öl |
| 55 | 2,4-$Cl_2$ | 3-Cl | -- | E | 2 | 73-77 |
| 56 | 2,4-$Cl_2$ | 3-$CF_3$ | -- | Z | 2 | Öl |
| 57 | 2,4-$Cl_2$ | 3-$CF_3$ | -- | E | 2 | 66-69 |
| 58 | 2,4-$Cl_2$-3-$CH_3$ | 4-Cl | -- | E/Z-Gemisch | 2 | Öl |
| 59 | 2,5-$Cl_2$ | 4-Cl | -- | Z | 2 | Öl |
| 60 | 2,5-$Cl_2$ | 4-Cl | -- | E | 2 | 82-86 |
| 61 | 2,4-$Cl_2$ | 2,4,6-$Cl_3$ | -- | Z-Isomer | 2 | 76-85 |
| 62 | 4-Cl | 2,4,6-$Cl_3$ | -- | Z-Isomer | 2 | 114-116 |
| 63 | H | 4-Cl | -- | Z-Isomer | 2 | 79-83 |
| 64 | H | 4-Cl | -- | Z-Isomer | 3 | 59-64 |
| 65 | H | 4-Cl | -- | Z-Isomer | 4 | 59-62 |
| 66 | H | 3-Cl | -- | Z-Isomer | 2 | 73-75 |
| 67 | H | 3-$CH_3$ | -- | Z-Isomer | 2 | 54-56 |
| 68 | H | H | .1/2 $CuCl_2$ | Z-Isomer | 4 | wachsartig |
| 69 | H | H | .HCl | E/Z-Gemisch | 4 | wachsartig |
| 70 | H | H | -- | E/Z-Gemisch | 4 | Öl |
| 71 | H | 4-Cl | .1/2 $CuCl_2$ | E/Z-Gemisch | 4 | wachsartig |
| 72 | H | 4-Cl | . HCl | E/Z-Gemisch | 4 | 108-115 |
| 73 | H | 4-Cl | -- | E/Z-Gemisch | 4 | Öl |
| 74 | H | 4-Cl | .1/2 $CuCl_2$ | E/Z-Gemisch | 2 | wachsartig |
| 75 | H | 4-Cl | . HCl | E/Z-Gemisch | 2 | 138-142 |
| 76 | H | 4-Cl | -- | E/Z-Gemisch | 2 | Öl |
| 77 | H | 3-Cl | -- | E/Z-Gemisch | 2 | Öl |
| 78 | H | 3-Cl | . HCl | E/Z-Gemisch | 2 | wachsartig |
| 79 | H | 3-Cl | .1/2 $CuCl_2$ | E/Z-Gemisch | 2 | wachsartig |
| 80 | H | 3-$CH_3$ | -- | E/Z-Gemisch | 2 | Öl |
| 81 | H | 3-$CH_3$ | . HCl | E/Z-Gemisch | 2 | wachsartig |
| 82 | H | 3-$CH_3$ | .1/2 $CuCl_2$ | E/Z-Gemisch | 2 | wachsartig |
| 83 | H | 4-$CH_3$ | -- | Z-Isomer | 2 | 110-112 |
| 84 | 4-$CH_3$ | 4-Cl | . HCl | Z-Isomer | 2 | 164-167 |
| 85 | 4-$CH_3$ | 3-Cl | -- | Z-Isomer | 2 | 86-91 |
| 86 | 4-$CH_3$ | 3-Cl | . HCl | Z-Isomer | 2 | Öl |
| 87 | 4-$CH_3$ | 3-$CH_3$ | -- | Z-Isomer | 2 | 66-71 |
| 88 | 4-$CH_3$ | 3-$CH_3$ | . HCl | Z-Isomer | 2 | 125-128 |

| | | | | | | |
|---|---|---|---|---|---|---|
| 89 | 4-$CH_3$ | 4-Cl | -- | Z-Isomer | 3 | 70-72 |
| 90 | 4-$CH_3$ | 4-Cl | . HCl | Z-Isomer | 3 | 160-163 |
| 91 | 4-$CH_3$ | H | -- | Z-Isomer | 4 | 58-61 |
| 92 | 4-$CH_3$ | H | . HCl | Z-Isomer | 4 | 120-124 |
| 93 | 4-$CH_3$ | 4-Cl | -- | Z-Isomer | 4 | 123-125 |
| 94 | 4-$CH_3$ | 4-Cl | . HCl | Z-Isomer | 4 | 123-125 |
| 95 | 4-Cl | 4-Cl | -- | Z-Isomer | 2 | 133-135 |
| 96 | 4-Cl | 4-Cl | . HCl | Z-Isomer | 2 | 174-178 |
| 97 | 4-Cl | 4-Cl | -- | Z-Isomer | 4 | 75-81 |
| 98 | 4-Cl | 4-Cl | . HCl | Z-Isomer | 4 | 128-130 |
| 99 | 4-Cl | H | -- | Z-Isomer | 4 | 51-56 |
| 100 | 4-Cl | H | . HCl | Z-Isomer | 4 | 136 |
| 101 | 4-Cl | 3-Cl | -- | Z-Isomer | 2 | 89-94 |
| 102 | 4-Cl | 3-Cl | . HCl | Z-Isomer | 2 | 141-145 |
| 103 | 4-Cl | 3-$CH_3$ | -- | Z-Isomer | 2 | 103-105 |
| 104 | 4-Cl | 3-$CH_3$ | . HCl | Z-Isomer | 2 | 107-108 |

**Tabelle**

B) Verbindungen der Formel (I) mit $R^2$ = $-(CH_2CH_2O)_uCH_2CH_2-$:

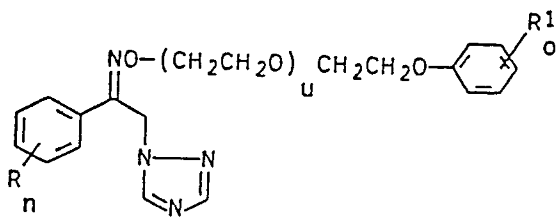

| Nr. | $R_n$ | $R_o^1$ | Konfiguration | u | Fp [°C] |
|---|---|---|---|---|---|
| 105 | 2,4-$Cl_2$ | H | E/Z-Gemisch | 1 | |
| 106 | 2,4-$Cl_2$ | H | E/Z-Gemisch | 2 | |
| 107 | 2,4-$Cl_2$ | H | E/Z-Gemisch | 3 | |
| 108 | 2,4-$Cl_2$ | 4-Cl | E/Z-Gemisch | 1 | |
| 109 | 2,4-$Cl_2$ | 4-Cl | E/Z-Gemisch | 2 | |
| 110 | 2,4-$Cl_2$ | 4-Cl | E/Z-Gemisch | 3 | |
| 111 | 2,4-$Cl_2$ | 2,4-$Cl_2$ | E/Z-Gemisch | 1 | |
| 112 | 2,4-$Cl_2$ | 2,4-$Cl_2$ | E/Z-Gemisch | 2 | |
| 113 | 2,4-$Cl_2$ | 2,4-$Cl_2$ | E/Z-Gemisch | 3 | |
| 114 | 2,4-$Cl_2$ | 2-Cl | E/Z-Gemisch | 2 | Öl |
| 115 | 2,4-$Cl_2$ | 3-Cl | E/Z-Gemisch | 2 | Öl |
| 116 | 2,4-$Cl_2$ | 4-$OCH_3$ | Z-Isomer | 2 | Öl |
| 117 | 2,4-$Cl_2$ | 2,4,6-$Cl_3$ | Z-Isomer | 2 | Öl |
| 118 | 2,4-$Cl_2$ | 2-Cl | E/Z-Gemisch | 1 | wachsartig |
| 119 | 2,4-$Cl_2$ | 4-$OCH_3$ | Z-Isomer | 1 | wachsartig |
| 120 | 2,4-$Cl_2$ | 3-$CH_3$ | Z-Isomer | 1 | wachsartig |
| 121 | 2,4-$Cl_2$ | 4-$CH_3$ | Z-Isomer | 1 | wachsartig |
| 122 | 4-Cl | 2,4,6-$Cl_3$ | Z-Isomer | 1 | 69-71 |
| 123 | 4-Cl | 2-Cl | Z-Isomer | 1 | 63-65 |
| 124 | 4-Cl | 3-$CH_3$ | Z-Isomer | 1 | 66-68 |
| 125 | 4-Cl | 4-$OCH_3$ | Z-Isomer | 1 | 71 |
| 126 | 4-Cl | 4-$CH_3$ | Z-Isomer | 1 | 79 |
| 127 | 4-Cl | H | Z-Isomer | 2 | Öl |
| 128 | 4-Cl | 4-Cl | Z-Isomer | 2 | 85-88 |
| 129 | 4-Cl | 2-Cl | Z-Isomer | 2 | Öl |
| 130 | 4-Cl | 3-Cl | Z-Isomer | 2 | Öl |
| 131 | 4-Cl | 2,4-$Cl_2$ | Z-Isomer | 2 | 48-53 |
| 132 | 4-Cl | 4-$OCH_3$ | Z-Isomer | 2 | Öl |
| 133 | 4-$CH_3$ | 4-Cl | Z-Isomer | 2 | Öl |
| 134 | H | 4-Cl | Z-Isomer | 2 | Öl |

Tabelle

C) Verbindungen der Formel (I) mit $R^2 = -(CH_2)_v-CH=CH-(CH_2)_t-$:

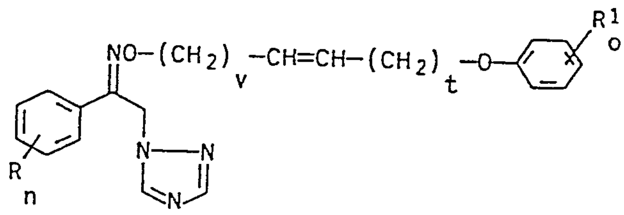

| Nr. | $R_n$ | $R_o^1$ | Konfiguration | v | t | Fp [°C] |
|---|---|---|---|---|---|---|
| 134a | 2,4-$Cl_2$ | H | E/Z-Gemisch | 1 | 1 | |
| 135 | 2,4-$Cl_2$ | 4-Cl | E/Z-Gemisch | 1 | 1 | |
| 136 | 2,4-$Cl_2$ | 4-$CH_3$ | E/Z-Gemisch | 1 | 1 | |
| 137 | 2,4-$Cl_2$ | 4-$OCH_3$ | E/Z-Gemisch | 1 | 1 | |
| 138 | 2,4-$Cl_2$ | 2,4-$Cl_2$ | E/Z-Gemisch | 1 | 1 | |
| 139 | 2,4-$Cl_2$ | 2,4,6-$Cl_3$ | E/Z-Gemisch | 1 | 1 | |
| 140 | 2,4-$Cl_2$ | 4-Cl | E/Z-Gemisch | 2 | 2 | |
| 141 | 2,4-$Cl_2$ | 4-Cl | E/Z-Gemisch | 3 | 3 | |
| 142 | 2,4-$Cl_2$ | 2-Cl | E/Z-Gemisch | 1 | 1 | 84 |

Tabelle

D) Verbindungen der Formel (I) mit $R^2 = -(CH_2)_v-C\equiv C-(CH_2)_t-$

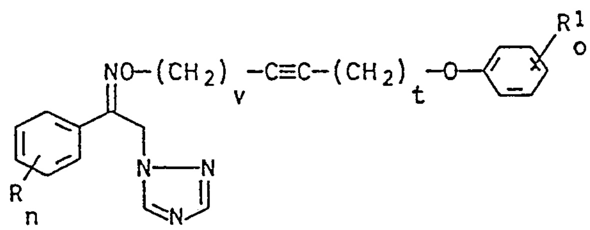

| Nr. | $R_n$ | $R_o^1$ | Konfiguration | v | t | Fp [°C] |
|---|---|---|---|---|---|---|
| 119 | 2,4-$Cl_2$ | H | E/Z-Gemisch | 1 | 1 | Öl |
| 120 | 2,4-$Cl_2$ | 4-Cl | E/Z-Gemisch | 1 | 1 | |
| 121 | 2,4-$Cl_2$ | 4-$CH_3$ | E/Z-Gemisch | 1 | 1 | Öl |
| 122 | 2,4-$Cl_2$ | 4-$OCH_3$ | E/Z-Gemisch | 1 | 1 | Öl |
| 123 | 2,4-$Cl_2$ | 2,4-$Cl_2$ | E/Z-Gemisch | 1 | 1 | Öl |
| 124 | 2,4-$Cl_2$ | 3-$CH_3$ | E/Z-Gemisch | 1 | 1 | Öl |
| 125 | 2,4-$Cl_2$ | 4-Cl | E/Z-Gemisch | 2 | 2 | |
| 126 | 2,4-$Cl_2$ | 4-Cl | E/Z-Gemisch | 3 | 3 | |
| 127 | 2,4-$Cl_2$ | 4-F | Z-Isomer | 1 | 1 | Öl |
| 128 | 2,4-$Cl_2$ | 3-Cl | Z-Isomer | 1 | 1 | Öl |
| 129 | 4-Cl | H | Z-Isomer | 1 | 1 | 70-75 |
| 130 | 4-Cl | 4-Cl | Z-Isomer | 1 | 1 | 81-85 |
| 131 | 4-Cl | 4-F | Z-Isomer | 1 | 1 | 70-75 |
| 132 | 4-Cl | 3-Cl | Z-Isomer | 1 | 1 | 80-85 |
| 133 | 4-Cl | 4-$OCH_3$ | Z-Isomer | 1 | 1 | Öl |

Charakteristische Daten ($^1$H-NMR):

| Nr. | δ in ppm, Lsgm. $CDCl_3$ |
|---|---|
| 13 | 4,05 "t" 2H; 4,45 "t" 2H; 5,39 s 2H, 7,72 s 1H; 7,98 s 1H |
| 14 | 3,90 "t" 2H; 4,25 "t" 2H; 5,10 s 2H; 7,82 s 1H; 7,90 s 1H |
| 15 | 4,00 "t" 2H; 4,35 "t" 2H; 5,40 s 2H; 7,80 s 1H; 8,07 s 1H |
| 16 | 3,90 "t" 2H; 4,20 "t" 2H; 5,20 s 2H; 7,90 s 1H; 8,00 s 1H |
| 17 | 4,00 "t" 2H; 4,30 "t" 2H; 5,44 s 2H; 7,82 s 1H; 8,10 s 1H |
| 18 | 3,90 "t" 2H; 4,20 "t" 2H; 5,20 s 2H; 7,93 s 1H; 8,04 s 1H |
| 32 | 4,03 "t" 2H; 4,43 "t" 2H; 5,44 s 2H; 7,80 s 1H; 8,07 s 1H |
| 33 | 3,90 "t" 2H; 4,27 "t" 2H; 5,20 s 2H; 7,90 s 1H; 8,00 s 1H |
| 35 | 3,78 "t" 2H; 4,08 "t" 2H; 5,05 s 2H; 7,70 s 1H; 7,85 s 1H |
| 36 | 3,38 "t" 2H; 4,20 "t" 2H; 5,35 s 2H; 7,70 s 1H; 7,98 s 1H |
| 39 | 4,22 "t" 2H; 4,48 "t" 2H; 5,23 s 2H; 7,93 s 1H; 8,03 s 1H |
| 49 | 4,25 "t" 2H; 4,60 "t" 2H; 5,47 s 2H; 7,80 s 1H; 8,17 s 1H |
| 54 | 4,27 "t" 2H; 4,60 "t" 2H; 5,48 s 2H; 7,80 s 1H; 8,15 s 1H |
| 56 | 4,43 "t" 2H; 4,63 "t" 2H; 5,45 s 2H; 7,80 s 1H; 8,13 s 1H |
| 58 | 2,41 s 3H; 4,22 "t" 2H; 4,58 "t" 2H; 5,41 s 2H; 7,78 s 1H; 8,11 s 1H |
| 59 | 4,22 "t" 2H; 4,59 "t" 2H; 5,42 s 2H; 7,81 s 1H; 8,14 s 1H |
| 119 | 4,78 s 4H; 5,22 s 2H; 7,93 s 1H; 8,03 s 1H |
| 121 | 2,28 s 3H; 4,70 m 3H; 5,20 s 2H; 7,90 s 1H; 8,00 s 1H |
| 122 | 3,72 s 3H; 4,70 s 2H; 4,87 s 2H; 5,44 s 2H; 7,80 s 1H; 8,11 s 1H |
| 123 | 4,70 m 4H; 5,20 s und 5,50 s 2H; 7,80 s und 7,94 s 1H; 8,01 s und 8,10 s 1H |
| 124 | 2,31 s 3H; 4,72 s 2H; 4,87 s 2H; 5,44 s 2H; 7,80 s 1H; 8,10 s 1H |
| 120 | 3,75 m 4H; 4,15 m 2H; 4,35 m 2H, 5,53 s 2H; 7,83 s 1H; 8,50 s 1H |
| 121 | 3,75 m 4H; 4,15 m 2H; 4,38 m 2H, 5,53 s 2H; 7,85 s 1H; 8,50 s 1H |
| 127 | 3,75 m 8H; 4,15 m 2H; 4,40 m 2H, 5,53 s 2H; 7,88 s 1H; 8,38 s 1H |
| 129 | 3,80 m 8H; 4,15 m 2H; 4,45 m 2H, 5,38 s 2H; 7,88 s 1H; 8,38 s 1H |
| 130 | 3,70 m 8H; 4,15 m 2H; 4,30 m 2H, 5,45 s 2H; 7,78 s 1H; 8,45 s 1H |
| 132 | 3,80 m 8H; 4,10 m 2H; 4,40 m 2H, 5,60 s 2H; 8,05 s 1H; 8,70 s 1H |
| 133 | 3,70 m 8H; 4,00 m 2H; 4,35 m 2H, 5,30 s 2H; 7,75 s 1H; 8,43 s 1H |
| 134 | 3,80 m 8H; 4,10 m 2H; 4,45 m 2H, 5,40 s 2H; 7,90 s 1H; 8,40 s 1H |
| 127 | 4,60 "t" 2H; 4,80 "t" 2H; 5,40 s 2H, 7,70 s 1H; 7,98 s 1H |
| 128 | 4,65 "t" 2H; 4,85 "t" 2H; 5,40 s 2H, 7,70 s 1H; 8,00 s 1H |
| 76 | 4,20 "t" 2H; 4,45 "t" 2H; 5,21 s 2H, 7,91 s 1H; 8,13 s 1H |
| 77 | 4,60 "t" 2H; 4,42 "t" 2H; 5,19 s 2H, 7,90 s 1H; 8,02 s 1H |
| 80 | 4,20 "t" 2H; 4,45 "t" 2H; 5,20 s 2H, 7,90 s 1H; 8,02 s 1H |
| 70 | 1,8 m 4H; 3,90 "t" 2H; 4,18 "t" 2H, 5,18 s 2H; 7,89 s 1H; 8,00 s 1H |
| 114 | 3,7 m 8H; 4,2 m 4H; 5,55 s 2H; 7,87 s 1H; 8,52 s 1H |
| 115 | 3,7 m 8H; 4,2 m 4H; 5,57 s 2H; 7,85 s 1H; 8,50 s 1H |
| 116 | 3,7 m 8H; 4,0 m 2H; 4,45 m 2H; 5,55 s 2H; 7,88 s 1H; 8,5 s 1H |
| 117 | 3,7 m 8H; 4,25 m 4H; 5,55 s 2H; 7,85 s 1H; 8,50 s 1H |
| 118 | 3,8 m 4H; 4,20 m 4H; 5,45 s 2H; 7,75 s 1H; 8,40 s 1H |
| 119 | 3,75 m 4H; 4,05 m 2H; 4,35 m 2H, 5,53 s 2H; 7,83 s 1H; 8,50 s 1H |
| 133 | 4,65 "t" 2H; 4,85 "t" 2H; 5,30 s 2H; 7,80 s 1H; 8,10 s 1H |

s: Singulett
t: Pseudotriplett

Die neuen Verbindungen und ihre Salze und Metallkomplexverbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum an Kürbisgewächsen,
Podosphaera leucotricha an Äpfeln,

Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Mycosphaerella musicola an Bananen,
Cercospora sojina an Soja und
Cercospora personata an Erdnüssen,
Hemileia vastatrix an Kaffee,
Pyricularia oryzae an Reis.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin, Dimethylformamid) und Wasser; Trägerstoffe wie natürliche Gesteinsmehle, z. B. Kaoline, Tonerden, Talkum, Kreide und synthetische Gesteinsmehle (z. B. hochdisperse Kieselsäure, Silikate); Emulgiermittel, wie nicht-ionogene und anionische Emulgatoren (z. B. Polyoxyethylen - Fettalkohol - Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,1 und 3 kg Wirkstoff oder mehr je ha.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

I. 20 Gewichtsteile der Verbindung Nr. 5 werden in 3 Gewichtsteilen des Natriumsalzes der Diisobutylnaphthalinsulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in Wasser erhält man eine Spritzbrühe.H

II. 3 Gewichtsteile der Verbindung Nr. 6 werden mit 97 Gewichtsteilen feinteiligem Kaolin innig vermischt. Man erhält auf diese Weise ein Stäubemittel, das 3 Gew.-% des Wirkstoffs enthält.

III. 30 Gewichtsteile der Verbindunng Nr. 12 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV. 40 Gewichtsteile der Verbindung Nr. 5 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit Wasser erhält man eine wäßrige Dispersion.

V. 20 Teile der Verbindung Nr. 6 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

VI. Man vermischt 90 Gewichtsteile der Verbindung Nr. 12 mit 10 Gewichtsteilen N-Methyl-alpha-pyrrolidon und erhält eine Lösung, die zur Anwendung in Form kleinster Tropfen geeignet ist.

VII. 20 Gewichtsteile der Verbindung Nr. 5 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

VIII. 20 Gewichtsteile der Verbindung Nr. 6 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IX. 10 Gewichtsteile der Verbindung Nr. 12, 20 Gewichtsteile Polyoxyethylensorbitan-monolaurat, 20 Gewichtsteile Methanol und 50 Gew.-Teile Wasser werden zu einer Lösung verrührt, die 10 Gew.-% des Wirkstoffs enthält. Durch Hinzufügen von weiterem Wasser können stärker verdünnte Lösungen hergestellt werden.

Die neuen Mittel können in diesen Aufwendungsformen auch zusammen mit anderen Wirkstoffen vorliegen, wie z. B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrößerung des Wirkungsspektrums. Bei einer Anzahl solcher Fungizidmischungen treten auch synergistische Effekte auf, d. h. die Wirksamkeit des Kombinationsproduktes ist größer als die addierten Wirksamkeiten der Einzelkomponenten.

Fungizide, die mit den neuen Verbindungen kombiniert werden können, sind beispielsweise:

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid
Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,

heterocyclische Strukturen, wie
2-Heptadecyl-2-imidazolin-acetat
2,4-Dichlor-6-(o-chloranilino)-s-triazin
0,0-Diethyl-phthalimidophosphonothioat
5-Amino-1-(bis-(dimethylamino)-phosphinyl)-3-phenyl-1,2,4-triazol)
2,3-Dicyano-1,4-dithiaanthrachinon
2-Thio-1,3-dithio-(4,5,6)-chinoxalin

1-(Butylcarbamoyl)-2-benzimidazol-carbaminsäuremethylester
2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethyl-phthalimid
N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Jod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin

1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1-H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
α-(2-Chlorphenyl)-α-(4-chlorphenyl)-5-pyrimidin-methanol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene Fungizide, wie
Dodecylguanidinacetat
3-(3-(3,5-Dimethyl-2-oxycyclohexyl)-2-hydroxyethyl)-glutaramid
Hexachlorbenzol
DL-Methyl-N-(2,6-dimethyl-phenyl)-N-furoyl(2)-alaninat,
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl(-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid.

Für die folgenden Versuche wurden die aus der DE-A-2 431 407 bekannten Verbindungen A 2,4-Dichlorphenyl-(1,2,4-triazolyl-1)-methylketon und B tertiär-Butyl-(1,2,4-triazolyl-1)-methylketon verwendet.

## Versuch 1

*Wirksamkeit gegen Weizenmehltau*

Blätter von in Töpfen gewachsenen Weizenkeimlingen der Sorte "Jubilar" werden mit wäßriger Spritzbrühe, die 80 % Wirkstoff und 20 % Emulgiermittel in der Trockensubstanz enthält besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 % relativer Luftfeuchtigkeit aufgestellt. Nach 7 Tagen wird das Ausmaß der Mehltauentwicklung ermittelt.

Das Ergebnis des Versuches zeigt, daß beispielsweise die Verbindungen 12, 18, 23, 40, 44, 49, 54 und 56 bei der Anwendung als 0,025, 0,006 oder 0,0015 %-ige Spritzbrühe eine bessere fungizide Wirkung (beispielsweise 97 %) zeigen als die bekannte Verbindung B (beispielsweise 60 %).

## Versuch 2

*Wirksamkeit gegen Botrytis cinerea an Paprika*

Paprikasämlinge der Sorte "Neusiedler Ideal Elite" werden, nachdem sich 4 bis 5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß beispielsweise die Verbindungen 12, 13 und 49 bei der Anwendung als 0,05 %-ige Spritzbrühe eine bessere fungizide Wirkung (beispielsweise 90 %) haben als die bekannte Verbindung A (beispielsweise 70 %).

## Versuch 3

*Wirksamkeit gegen Weizenbraunrost*

Blätter von in Töpfen gewachsenen Weizensämlingen der Sorte "Jubilar" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschließend mit wäßrigen Spritzbrühen, die 80 % Wirkstoff und 20 % Emulgiermittel in der Trockensubstanz enthalten, tropfnaß gespritzt. Nach dem Antrocknen des Spritzbelages werden die Versuchspflanzen im Gewächshaus bei Temperaturen zwischen 20 und 22°C und 65 bis 70 % relativer Luftfeuchtigkeit aufgestellt. Nach 8 Tagen wird das Ausmaß der Rostpilzentwicklung auf den Blättern ermittelt.

Das Ergebnis des Versuchs zeigt, daß beispielsweise die Verbindungen 17, 23, 34, 36 und 44 bei der Anwendung als 0,025 oder 0,006 %-ige Spritzbrühe eine bessere fungizide Wirkung (beispielsweise 97 %) haben als die bekannte Verbindung A (70 %).

## Patentansprüche

1. Azolylacetophenonoximether der Formel (I)

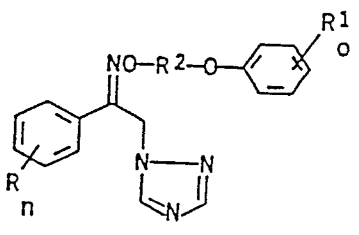

in welcher
R und $R^1$ unabhängig voneinander Wasserstoff, Halogen, $C_1$-$C_4$-Alkyl, $C_1$-$C_2$-Alkoxy, $C_1$-$C_2$-Alkylthio, $C_1$-$C_4$-Alkylsulfonyl, $C_1$-$C_4$-Halogenalkyl mit bis zu 5 Halogenatomen, Nitro, Cyano, Phenyl, Phenoxy oder durch Halogen, Cyano, Nitro oder $C_1$-$C_2$-Halogenalkyl mit bis zu 3 gleichen oder verschiedenen Halogenatomen substituiertes Phenyl oder Phenoxy bedeuten,
$R^2$ ein Diradikal der Formel $-(CH_2)_m-$ oder der Formel $-(CH_2CH_2O)_uCH_2CH_2-$ oder der Formel $-(CH_2)_v-CH{=}CH-(CH_2)_t-$ oder der Formel $-(CH_2)_v-C{\equiv}C-(CH_2)_t-$ bedeutet,
n, o, u, v, t unabhängig voneinander die Zahlen 1, 2 oder 3 bedeuten,
m die ganzen Zahlen von 2 bis 8 bedeutet
und deren Salze und Komplexverbindungen.

2. Fungizid, *gekennzeichnet durch* einen Gehalt an einem Trägerstoff und einem Azolylacetophenoximether gemäß Anspruch 1.

3. Fungizid, *gekennzeichnet durch* einen Gehalt an einem Trägerstoff und einem Z-Isomeren eines Azolylacetophenonoximethers gemäß Anspruch 1.

4. Fungizid, *gekennzeichnet durch* einen Gehalt an einem Trägerstoff und einem E-Isomeren eines Azolylacetophenonoximethers gemäß Anspruch 1.

5. 2,4-Dichlor-ω-[1,2,4-triazolyl-(1)]-acetophenon-oxim-0-2-(4-chlorphenoxy)-ethylether.

6. 2,4-Dichlor-ω-[1,2,4-triazolyl-(1)]-acetophenon-Z-oxim-0-2-(4-chlorphenoxy)-ethylether.

7. 2,4-Dichlor-ω-[1,2,4-triazolyl-(1)]-acetophenon-oxim-0-2-(2,4,6-trichlorphenoxy)-ethylether.

8. 2,4-Dichlor-ω-[1,2,4-triazolyl-(1)]-acetopnenon-Z-oxim-0-2-(4-fluorphenoxy)-ethylether.

9. 2,4-Dichlor-ω-[1,2,4-triazolyl-(1)]-acetophenon-oxim-0-5-phenoxypentylether.

10. Verfahren zur Bekämpfung von Pilzen, *dadurch gekennzeichnet*, daß man diese oder die vor Pilzbefall zu schützenden Saatgüter, Pflanzen oder den Boden mit einem Azolylacetophenonoximether gemäß Anspruch 1 behandelt.

## Claims

1. An azolylacetophenone oxime ether of the formula (I)

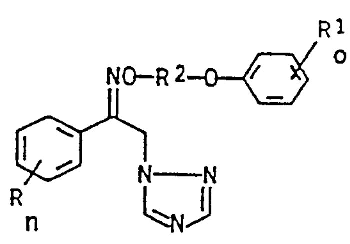

where R and $R^1$ independently of one another are each hydrogen, halogen, $C_1$-$C_4$-alkyl, $C_1$ or $C_2$-alkoxy, $C_1$ or $C_2$-alkylthio, $C_1$-$C_4$-alkylsulfonyl, $C_1$-$C_4$-haloalkyl containing not more than 5 halogen atoms, nitro, cyano or phenyl or phenoxy, either of which is unsubstituted or substituted by halogen, cyano, nitro or $C_1$ or $C_2$-haloalkyl containing not more than 3 identical or different halogen atoms,

$R^2$ is a divalent radical of the formula $-(CH_2)_m-$, $-(CH_2CH_2O-)_uCH_2CH_2-$, $-(CH_2)_v-CH{=}CH-(CH_2)_t-$ or $-(CH_2)_v-C{\equiv}C-(CH_2)_t-$, n, o, u, v and t independently of one another are each 1, 2 or 3, m is an integer from 2 to 8, or a salt or metal complex thereof.

2. A fungicide containing a carrier and an azolylacetophenoxime ether as claimed in claim 1.

3. A fungicide containing a carrier and a Z isomer of an azolylacetophenone oxime ether as claimed in claim 1.

4. A fungicide containing a carrier and an E isomer of an azolylacetophenone oxime ether as claimed in claim 1.

5. 2,4-Dichloro-ω-[1,2,4-triazol-1-yl]-acetophenone oxime-0-2-(4-chlorophenoxy)-ethyl ether.

6. 2,4-Dichloro-ω-[1,2,4-triazol-1-yl]-acetophenone-Z-oxime-0-2-(4-chlorophenoxy)-ethyl ether.

7. 2,4-Dichloro-ω-[1,2,4-triazol-l-yl]-acetophenone oxime-0-2-(2,4,6-trichlorophenoxy)-ethyl ether.

8. 2,4-Dichloro-ω-[1,2,4-triazol-1-yl]-acetophenone-Z-oxime-0-2-(4-fluorophenoxy)-ethyl ether.

9. 2,4-Dichloro-ω-[1,2,4-triazol-1-yl]-acetophenone oxime-0-5-phenoxypentyl ether.

10. A process for combating fungi, which comprises treating the fungi or the seed, plants or soil to be protected against fungus attack with an azolylacetophenone oxime ether as claimed in claim 1.

## Revendications

1. Azolylacetophenonoximether de formule (I)

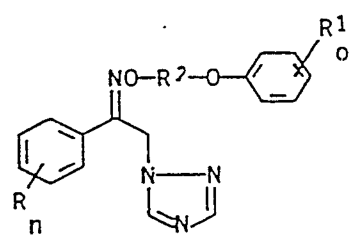

dans laquelle

R et $R^1$ représentent, indépendamment l'un de l'autre, hydrogène, halogène, alkyle en $C_1$-$C_4$, alcoxy en $C_1$-$C_2$ alkylthio en $C_1$-$C_2$, alkylsulfonyle en $C_1$-$C_4$, halogénalkyle en $C_1$-$C_4$ ayant jusqu'à 5 atomes d'hydrogène nitro, cyano, phényle, phenoxy ou phényle substitué par halogène, cyano, nitro ou halogénalkyle en $C_1$-$C_2$ ayant jusqu'à 3 atomes d'halogène identiques ou différents.

$R^2$ représente un diradical de formule $-(CH_2)_m$ ou de formule $-(CH_2-CH_2O)\ CH_2CH_2-$ ou de formule $-(CH_2)_v-CH{=}CH-(CH_2)_t-$ ou de formule $-(CH_2)_v-C{\equiv}C-(CH_2)_t$

n, o, u, v, t, représentent indépendamment l'un de l'autre les nombres 1, 2 ou 3

m représente les nombres entiers de 2 à 8, et ses sels et composés complexes.

2. Fongicide, caractérisé par une teneur en une matière de support et un azolylacetophenonoximether selon la revendication 1.

3. Fongicide, caractérisé par une teneur en une matière de support et un isomére Z d'un azolacetophenonoximether selon la revendication 1.

4. Fongicide, caractérisé par une teneur en une matière de support et un isomére E d'un azolylacetophenonoximether selon la revendication 1.

5. 2,4-Dichloro-ω-[1,2,4-triazolyl-(1)]acétophenon-oxim-0-2-(4-chlorphenoxy)-ethylether.

6. 2,4-Dichloro-ω-[1,2,4-triazolyl-(1)]-acétophenon-Z-oxim-0-2-(4-chlorphenoxy)-ethylether.

7. 2,4-Dichloro-ω-[1,2,4-triazolyl-(1)]-acétophenon-oxim-0-2-(2,4,6-trichlorphenoxy)-ethylether.

8. 2,4-Dichloro-ω-[1,2,4-triazolyl-(1)]-acétophenon-Z-oxim-0-2-(4-fluorphenoxy)-ethylether.

9. 2,4-Dichloro-ω-[1,2,4-triazolyl-(1)]-acétophenon-oxim-0-5-phenoxy-pentylether.

10. Procédé de lutte contre les champignons, caractérisé par le fait que l'on traite ceux-ci ou les semences, plantes ou sols à protéger contre l'attaque par champignons, avec de l'azolylacetophenonoximether selon la revendication 1.